# EUROPÄISCHE PATENTANMELDUNG

(11) **EP 0 707 831 A1**
(43) Veröffentlichungstag der Anmeldung: **24.04.1996**
(21) Anmeldenummer: 95115962.3
(22) Anmeldetag: 10.10.1995
(51) Int. Cl.: A61B 17/22

(54) **Vorrichtung zum Abtragen von Gewebematerial in intrakorporalen Hohlkanälen mit einem Laserkatheter**

(30) Priorität: 20.10.1994 DE 4437578
(71) Anmelder: MEDOLAS Gesellschaft für Medizintechnik, D-92224 Amberg (DE)
(72) Erfinder: Erbel, Raimund, Prof., D-45239 Essen (DE); Koch, Lothar, Dr., D-55130 Mainz (DE); Roth, Thomas, Dr., D-65187 Wiesbaden (DE)
(74) Vertreter: Münich, Wilhelm, Dr.

(57) **Zusammenfassung**

Beschrieben wird eine Vorrichtung zum Abtragen von Gewebematerial in intrakorporalen Hohlkanälen mit einem Laserkatheter, der distalseitig einen Raumbereich vorsieht, in den das abzutragende Gewebematerial einbringbar ist, das mit Hilfe von längs zur Katheterachse beweglichen Lichtleitfasern mittels Lichtapplikation vom übrigen Gewebe abtrennbar ist, die nach dem Abtrennvorgang den Raumbereich abschließen.

Die Erfindung zeichnet sich dadurch aus, daß der Laserkatheter ein Hohlkatheter ist, durch dessen inneren Hohlkanal eine Diagnoseeinheit durchführbar ist.

## Beschreibung

Die Erfindung bezieht sich auf eine Vorrichtung zum Abtragen von Gewebematerial in intrakorporalen Hohlkanälen mit einem Laserkatheter, der distalseitig einen Raumbereich vorsieht, in den das abzutragende Gewebematerial einbringbar ist, das mit Hilfe von längs zur Katheterachse beweglichen Lichtleitfasern mittels Lichtapplikation vom übrigen Gewebe abtrennbar ist, die nach dem Abtrennvorgang den Raumbereich abschließen.

Zur Beseitigung von Verengungen und Verschlüssen in intrakorporalen Hohlkanälen, wie z. B. koronare oder periphere Gefäße, werden heute die verschiedensten Techniken angewandt. In den koronaren und peripheren Gefäßen wird die am weitesten verbreitete Technik der Ballondilatation angewendet. Mit Hilfe von inflatierbaren Ballonhüllen, die an die Stellen der Gefäßverengung positioniert werden, werden die Gewebeablagerungen, die sogenannten Stenosen derart zur Seite gedrückt, so daß der Gefäßdurchmesser wieder vergößert werden kann. Dies wird in manchen Fällen durch Einsetzen eines Drahtgeflechts unterstützt, wovon man sich bessere Langzeitergebnisse hinsichtlich der Wiederverschlußrate bzw. Wiederverengungsrate erhofft.

Die Verringerung der Wiederverengungs- bzw. Verschlußrate ist auch das Ziel weiterer Techniken zur Wiedereröffnung von Arterien. Der sogenannte Rotablator, eine sich schnell drehende Diamantspitze, dient als Fräser im Gefäß, der das Stenosematerial pulverisiert.

Alternativ dazu wird stenosierendes Material mit Hilfe des Einsatzes von Laser ablatiert. Die hierbei entstehenden Fragmete sind nicht größer als die Zellstrukturen des Blutes und stellen somit im weiteren Verlauf der Gefäße keine Gefahr dar.

Auf dem Gebiet der Angioplastie, d. h. die minimal invasive Gefäßchirugie, hat sich der Excimer-Laser mit seiner UV-Strahlung am besten bewährt.

Mit diesen Techniken ist es zwar möglich vor Ort Stenosematerial abzutragen, doch verbleiben die abgetragenen Bruchstücke auf diese Weise im Körper. Gleichwohl der intrakorporale Verbleib kleinster Stücke an Gewebematerial weitgehen ungefährlich ist, sind Anwendungen denkbar, bei denen eine Gewebeanalyse von Stenosematerial sehr wünschenswert ist.

Aus der EP 0 163 502 A3 ist eine Katheteranordnung bekannt, die das, das Gefäß verengende Material mittels einer Schneidevorrichtung abtrennt und das abgetrennte Material im Katheterinneren samt Katheter extrakorporal verbringt. Nachteilhaft bei dieser Anordnung ist jedoch die Gewebeverspannung, die das Gewebe während des Schneidevorganges erfährt. Traumatische Gewebeirritationen können durch die mechanische Belastung die Folge sein.

Zur Verrigerung des mechanischen Zuges an der Arterienwand durch das Messer des vorgenannten Artherektomiekatheters wird in der EP 0 411 496 A1 die Kombination des Katheters mit einem Laser vorgeschlagen. Hierbei wird das Messer durch Lichtleitfasern ersetzt, die, mit einem aktivierten Laser verbunden, das in die Kammer des Katheters hineinragende Material ohne mechanische Belastung der Gefäßwand schneiden.

Beiden Verfahren ist jedoch folgende Problematik gemein:
Die Erfolgskontrolle über die Güte und Vollständigkeit des Gewebeabtrages kann nur nach bereits vollendetem Gewebeabtrag mit Hilfe einer angiographischen Beobachtung des Gefäßes erfolgen. So ist es während des Eingriffes nicht möglich genau zu beurteilen, wieviel Stenosematerial in die Katheterkammer zur Aufnahme des Gewebematerials hineinragt und wieviel tatsächlich abgetrennt worden ist. Dies hat zur Folge, daß Wiederholungen der Schneideprozedur notwendig sind. Zwischen jedem dieser Schneidprozesse muß der Katheter für die angiographische Kontrolle zurückgezogen werden. Dies und die notwendige erneute Plazierung des Katheters ist mit einer erhöhten Belastung durch Röntgenstrahlen und Kontrastmittel des Patienten verbunden. Hinzu kommt, daß durch häufige Bewegung des Katheters die Verletzungsgefahr für die Gefäßwand gesteigert wird.

Zudem sind beide Verfahren nur für stark exzentrische Stenosen in großen Gefäßen geeignet, da der Artherektomiekatheter ein vergleichbar großes und unflexibles Instrument ist.

Der Erfindung liegt die Aufgabe zugrunde eine Vorrichtung zur Beseitigung von Verengungen und Verschlüssen in intrakorporalen Hohlkanälen anzugeben, die eine direkte intraluminale Kontrolle ermöglicht, so daß die Prozedur schneller und effizienter durchgeführt werden kann. Mit einer solchen direkten intraluminalen Kontrolle ist eine angiographische Beobachtung, die u.U. mit einer Röntgenstrahlenbelastung des Patienten verbunden ist, nur noch für die erste Plazierung des Katheters und die abschließende Kontrolle notwendig, so daß die Belastung des Patienten durch Röntgenstrahlung und Kontrastmittel wesentlich reduziert werden soll.

Des weiteren soll die Vorrichtung eine höhere Flexibilität als die bekannten Systeme aufweisen und somit auch für stärker gewundene sowie kleinere Gefäße geeignet sein.

Die Lösung der Aufgabe ist im Anspruch 1 angegeben. Vorteilhafte Ausführungsformen sind Gegenstand der Ansprüche 2ff..

Erfindungsgemäß ist eine Vorrichtung zum Abtragen von Gewebematerial in intrakorporalen Hohlkanälen mit einem Laserkatheter, der distalseitig einen Raumbereich vorsieht, in den das abzutragende Gewebematerial einbringbar ist, das mit Hilfe von längs zur Katheterachse beweglichen Lichtleitfasern mittels Lichtapplikation vom übrigen Gewebe abtrennbar ist, die nach dem Abtrennvorgang den Raumbereich abschließen, derart ausgestaltet, daß der Laserkatheter ein Hohlkatheter ist, durch dessen inneren Hohlkanal eine Diagnoseeinheit durchführbar ist.

Der Erfindung liegt die Idee zu Grunde die Gewebeentnahme mit Hilfe eines an sich bekannten Laserartherektomiekatheters mit einer Einrichtung zu versehen, die eine in-situ Kontrolle des Abtragevorganges ermöglicht. Hierzu muß der Laserkatheter grundsätzlich als Hohlkatheter ausgebildet sein, durch dessen inneren Kanal eine entsprechende Diagnoseeinheit oder eine Therapieeinheit durchführbar ist. Dies sind in vorteilhafter Weise ein flexibles Sichtendoskop, ein Angioskop, ein Ultraschallkatheter oder auch andere Untersuchungssysteme.

Der erfindungsgemäße Laserkatheteter besteht im wesentlichen aus einem flexiblen Schlauch, in dessen Inneren ein Ringkatheter geführt ist, der ebenfalls einen schlauchartigen, flexiblen Hohlkanal mit zirkular um diesen angeordnete Lichtleitfasern vorsieht. Der distale Endbereich des Katheters ist bevorzugt mit einem Kapselgehäuse versehen, das im wesentlichen die Form eines Hohlzylinders aufweist und etwa bis zur Hälfte ihres Umfanges in axialer Erstreckung eine trapezförmige Ausnehmung vorsieht. Eine in den Katheter eingebrachte Diagnoseeinheit vermag nun durch die Ausnehmung im Kapselgehäuse zu blicken, so daß eine Vor-Ort-Beobachtung der Gewebverhältnisse möglich ist.

Das Kapselgehäuse weist eine Katheterkammer auf, in die das abzutrennede Stenosematerial einbringbar ist. Die genaue Feinausrichtung der offenen Katheterkammer realtiv zur Stenose erfolgt erfindungsgemäß unter Beobachtung mit Hilfe der eingebrachten Diagnoseinheit, so daß zumindest für diese Justierarbeiten auf, den Patienten belastende Beobachtungsmethoden, wie bspw. Röntgenuntersuchung, verzichtet werden kann.

Um die Flexibilität am distalen Endbereich weiter zu erhöhen ist erfindungsgemäß eine Katheterspitze vorgesehen, die einen Katheterraum vorsieht, der durch den Zwischenraum zweier voneinander beabstandeter Ringe beschrieben ist. Durch diese erfindungsgemäße Ausführungsform wird das Prinzip einer weitgehend starren Katheterspitze verlassen, um Untersuchungen an noch filigraneren Blutgefäßen, bspw. Koronarverästelungen am Herzen, zu ermöglichen.

Die Erfindung wird nachstehend ohne Beschränkung des allgemeinen Erfindungsgedankens anhand von Ausführungsbeispielen unter Bezugnahme auf die Zeichnungen exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig.1: Vorrichting zur Beseitigung von Verengungen und Verschlüssen in intrakorporalen Hohlkanälen unter direkter intraluminaler Kontrolle in einer ersten Ausführungsform entlang eines Längsschnitts,
- Fig. 2: Querschnitt durch die erfindungsgemäße Vorrichtung zur besonderen Hervorhebung des Ringkatheters in dieser Ausführungsform,
- Fig. 3: Vorrichting zur Beseitigung von Verengungen und Verschlüssen in intrakorporalen Hohlkanälen unter direkter intraluminaler Kontrolle in der flexibleren Ausführungsform in der Gesamtansicht,
- Fig. 4a: einen Längsschnitt durch die erfindungsgemäße Vorrichtung,
- Fig. 4b: Querschnitt durch die erfindungsgemäße Vorrichtung zur besonderen Hervorhebung des Fasertkatheters,
- Fig. 4c: Querschnitt durch die erfindungsgemäße Vorrichtung zur besonderen Hervorhebung des distalen Ringes,
- Fig. 4d: Querschnitt durch die erfindungsgemäße Vorrichtung zur besonderen Hervorhebung des proximalen Ringes mit Fasertkatheter,
- Fig. 4e: Querschnitt durch die erfindungsgemäße Vorrichtung zur besonderen Hervorhebung des flexiblen Hohlkanals mit Fasertkatheter und
- Fig. 4f: eine Detaillvergrößerung zur besonderen Hervorhebung der Verbindung des Faserkatheters mit dem distalen Ring.

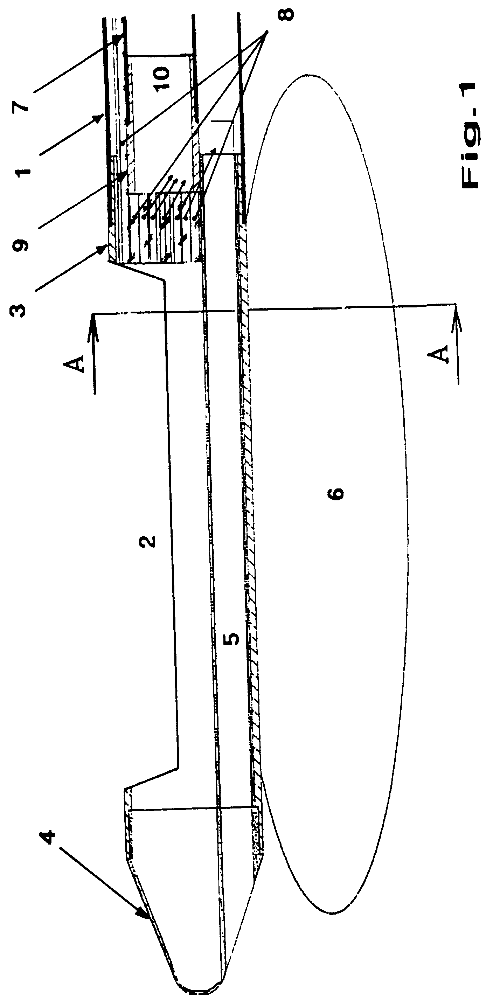

Figur 1 stellt den erfindungsgemäßen Laserkatheter dar, der am distalen Ende eines flexiblen Schlauches 1 eine Katheterkammer 2 vorsieht, die aus einer Hülse 3 mit einer seitlichen Ausnehmung besteht. Die, die Hülse abschließende Spitze 4 ist ebenfalls hohl und kann abgetrenntes Stenosematerial aufnehmen. Der flexible Schlauch 1, die Hülse 3 und die Spitze 4 bilden zusammen den Hüllkatheter. Zur sicheren Führung der Vorrichtung im Gefäß ist für die Aufnahme eines Führungsdrahtes ein zusätzliche Hohlkanal 5 vorgesehen. Zur Plazierung der Vorrichtung realtiv zur Stenose ist an der Unterseite der Katheterkammer 2 ein Ballon 6 angebracht.

Die Hülse 3 der Katheterkammer 2 besteht in vorteilhafter Weise aus Metall oder aus einem geeigneten, etwas flexiblen Material. Letzteres ermöglicht eine Anpassung an die Krümmung der Arterie und somit eine leichtere Plazierung der Vorrichtung.

Innerhalb des Hüllkatheters befindet sich ein Ringkatheter, der aus einem Schlauch 7 und an seinem Außenumfang ringförmig angeordnete Lichtleitfasern 8 besteht. Der Ringkatheter ist weitgehend flexibel, so daß das Einführen des Katheters in kleinste Gefäßwindungen möglichst schonend erfolgen kann. Am distalen Ende des Schlauches 7 ist ein Versteifungstück 9 eingesetzt, damit zumindest die Licht-Austrittsbereiche der Lichtleitfasern 8 eine möglichst feste gegenseitige Zuordnung erhalten. Das Versteifungsstück 9 ist aus steifem Material und an die Außengeometrie des Schlauches 7 derart angepaßt, so daß die Lichtleitfasern 8 übergangslos an dem Außenumfgang des Versteifungsstücks 9 anliegen.

Der Ringkatheter weist einen inneren Hohlkanal 10 auf, über dessen distales Ende die Lichtleitfasern 9 leicht hinausragen. Der Außendurchmesser der ringförmig angeordneten Lichtleitfasern 8 ist dabei annähernd so groß bemessen wie der Innendurchmesser der Hülse 3 der Katheterkammer 2, so daß der Ringkatheter, d.h. die Lichtleitfasern 8 zusammen mit dem Schlauch 7 und dem Versteifungsstück 9 axial innerhalb der Katheterkammer bewegbar sind.

Wie dem Ausführungsbeispiel in Figur 1 zu entnehmen ist, erstrecken sich die Lichtleitfasern 8 nicht über den gesamten Umfang des Schlauches 7 sowie des Versteifungsstücks 9, sondern nur über die obere Hälfte des Ringkatheters. Somit ist auf jeden Fall gewährtleistet, daß die Ausnehmung der Katheterkammer 2 von den Lichtleitfasern 8 nach Außen hin abgeschlossen ist.

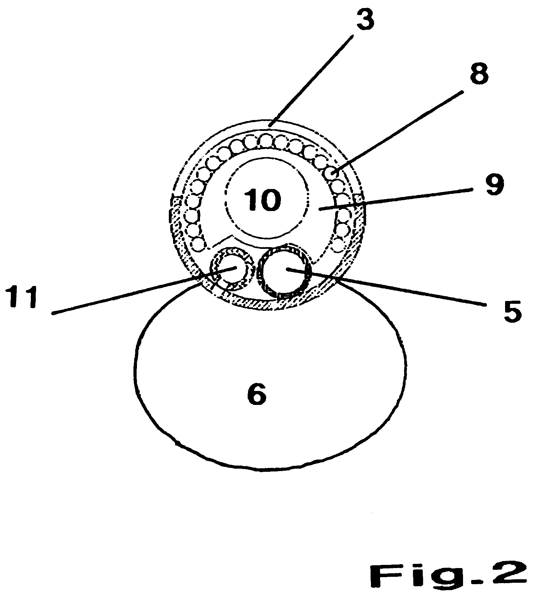

Zur sicheren Führung der gesamten Katheteranordnung durch die Gefäßkanäle wird ein Führungsdraht in die Gefäße eingeführt, über den nach entsprechender intrakorporaler Positionierung die gesamte Katheteranordnung zu schieben ist. Hierzu weist die Ausführungsform gemäß Figur 1 im Inneren des Hüllkatheters einen Führungskanal 5 auf, durch den der Führungsdraht (nicht in Figur 1 dargestellt) zu führen ist. Der Hohlkanal 5 zur Aufnahme des Führungsdrahtes ist im unteren Bereich des Hüllkatheters zusammen mit einem weiteren Hohlkanal durch den der Ballon 6 inflatierbar ist, vorgesehen. Gemäß Figur 2 sind die Kanäle 5 und 11 derart angeordnet, daß der Ringkatheter durch eine entsprechende Ausformung am distalen Ende des Versteifungstsückes 9 innerhalb des Hüllkatheters auf den Hohlkanälen 5 und 7 geführt werden kann.

Durch den inneren Hohlkanal 10 ist wahlweise ein Angioskop oder ein Ultraschallkopf zu diagnostischen Zwecken bis an die zu behandelnde Stelle der Arterie einbringbar.

An der Unterseite der Katheterkammer 2 ist zudem ein Ballon 6 befestigt, der über den Hohlkanal 11 und dem Verbindungskanal mit Kochsalzlösung oder Kontrastmittel befüllbar ist.

Diese Ausführungsform zeigt gegenüber dem bisherigen Stand der Technik in der Praxis entscheidende Vorteile:
Der Katheter wird unter Röntgenkontrolle mit vorgeschobenen Faserkatheter und somit geschlossener Kammer an die Stelle der Stenose plaziert. Am Stenoseort wird der Faserkatheter zurückgezogen und die Kammer geöffnet. Die genaue Ausrichtung erfolgt durch Drehen des Katheters unter gleichzeitiger angioskopischer Beobachtung mittels eines durch den inneren Hohlkanal vorgeschobenes Angioskop. Ist Plaque- bzw. Stenosenmaterial zu sehen, so wird die offene Seite der Katheterkammer durch Aufblasen des Ballons über dieses Plaquematerial geführt. Das Ausmaß des in die Kammer hineinragenden Materials wird unter Sichtkontrolle bzw. Ultraschallkontrolle durch den Inflationsdruck im Ballon gesteuert. Durch Vorschieben des Ringkatheter, der mit seinem proximalen Ende mit einem Laser gekoppelt ist, und unter gleichzeitiger Aktivierung des Lasers wird das in die Kammer hineinragende Plaquematerial abgetrennt. Hierbei muß mit dem Ringkatheter nur ein mäßiger mechanischer Druck auf das abzutrennende Material ausgeübt werden, so daß dieses nicht ausweicht. Bei vorgeschobenem Ringkatheter ist die Kammer geschlossen und das abgetrennte Material geborgen.

Die Erfolgskontrolle erfolgt über das sich im inneren Hohlkanal befindende Angioskop. Dieses ist problemlos zu entfernen und wieder zu plazieren, ohne den ganzen Katheter in seiner Position zu verändern. Dadurch kann nach Entfernen des Angioskops das abgetrennte Material abgesaugt und extrakorporal verbracht werden.

Alternativ ist ebenso ein Angioskop mit Arbeitskanal zu verwenden, durch den das Material mit Hilfe eines mechanischen Instruments entfernbar ist. Dieser Prozeß ist durch Spülflüssigkeit, die durch den äußeren Schlauch eingebracht wird, zu unterstützen. Alternativ dazu ist es möglich, das abgetrennte Material in die vordere, hohle Spitze der Kammer zu schieben und dort sicher zu lagern.

Die Prozedur kann so mehrfach wiederholt werden ohne daß der Katheter jedesmal neu plaziert werden muß. Eine Plazierung des Katheters in eine andere anguläre Position an gleicher Stelle des Gefäßes (z. B. von 1 auf 6 Uhr-Position) erfolgt über die angioskopischer Kontrolle, so daß keine erhöhte Strahlenbelastung für den Patienten auftritt.

Die Kontrolle kann auch durch Ultraschallbeobachtung erfolgen. Hierbei wird die geöffnete Seite der Kammer unter Ultraschallkontrolle zur Stenose ausgerichtet und mit Hilfe des Ballons an die Stenose herangedrückt. Das in die Kammer hineinragende Material wird wie oben beschrieben abgetrennt und anschließend abgesaugt oder in die Spitze verbracht. Die Erfolgskontrolle erfolgt anschließend mit Ultraschall und geöffneter Kammer.

Zur spektroskopischen Untersuchung der abgetrennten Materialien in der Kammer können durch den inneren Hohlkanal weitere Lichtleitfasern in die Kammer geführt werden. Durch einen Teil dieser Fasern kann Laserlicht beliebiger Wellenlänge zur optischen Anregung an das Material gebracht werden, die übrigen Lichtleitfasern übertragen die Fluoreszenzstrahlung zu einer proximalseitig vorgesehenen, spektroskopischen Untersuchungseinheit. Diese Technik sind beispielsweise im Europäischen Patent EP 0 3 116 658 und US Patent Nr. 4967745 dargestellt.

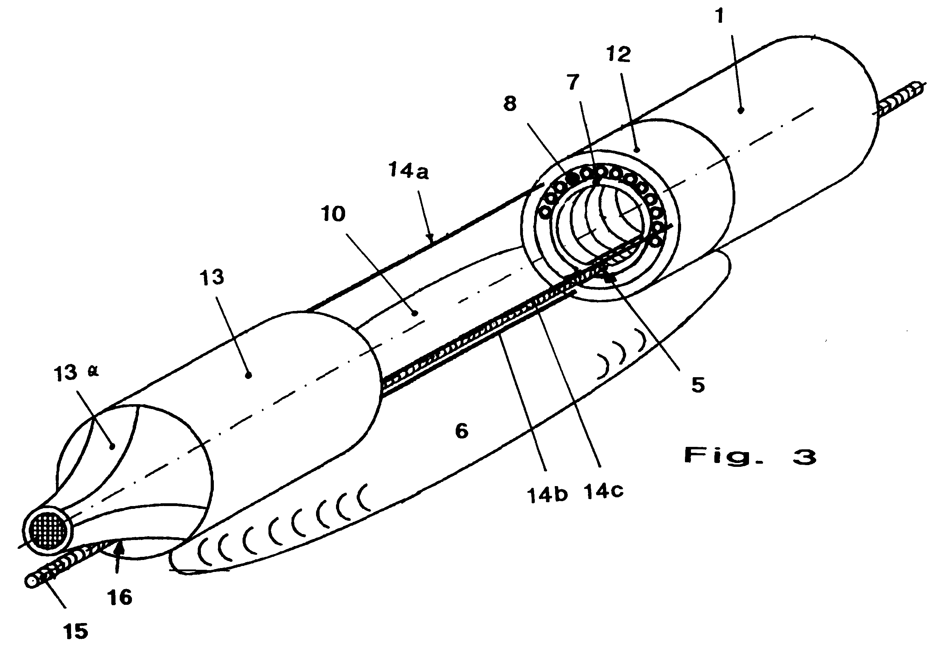

Aus der Figur 3 ist eine weitere erfindugsgemäße Ausführungsform der Katheterspitze mit erhöhter Flexibilität wiedergegeben. Hierbei wird die Hülse des Hüllkatheters durch zwei Ringe 12 und 13 ersetzt, die flexibel miteinander in einem bestimmten Abstand verbunden sind. Diese flexible Verbindung besteht vorzugsweise aus drei Drähten 14 a-c. Zusätzlich oder alternativ sind auch Bandverbindungen vorgesehen, die insbesondere eine leichtere Anbringung des Ballons 6 an dem distalen Bereich des Katheters ermöglichen. Der Hüllkatheter 1 ist distalseitig mit dem Ring 12 verbunden und umschließt, wie auch in der vorstehend beschriebenen Ausführungsform den Ringkatheter, der aus einem flexiblen Schlauch 7 sowie den Lichtleitfasern 8 besteht.

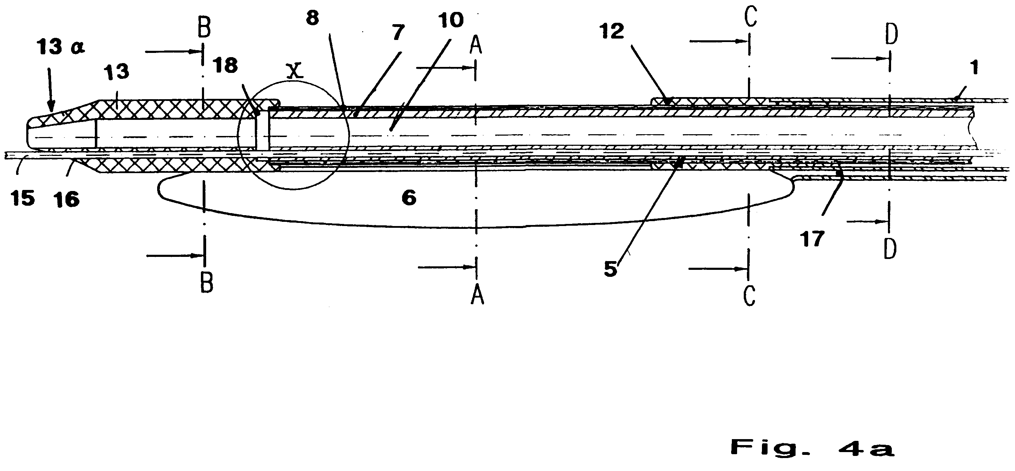

Zur sicheren Führung des Katheters im Gefäß ist ebenso ein Führungsdraht 15 vorgesehen, dessen Verlauf im Distalbereich des Laserkatheters in einer Querschnittsdarstellung aus Figur 4a hervorgeht. Der Führungsdraht 15 verläuft im Unterschied zur vorstehend beschriebenen Ausführungsform in einem Hohlkanal, der innerhalb des Ringkatheters selbst vorgesehen ist, d.h. der Hohlkanal 5 verläuft innerhalb des Schlauches 7 oder ist alternativ dazu im Mantelbereich des Schlauches 7 eingearbeitet. Desweiteren ist eine Durchführungsöffnung 16 für den Führungsdraht 15 ebenso im distalen Ring 13 vorgesehen, die von der axialen Lage mit dem Hohlkanal innerhalb des Ringkatheters fluchtet. Auf diese Weise ist sichergestellt, daß der Führungsdraht 15 eine exakte axiale Verschiebung des Ringkatheters zwischen beiden Ringteilen 12 und 13 gewährleistet.

An der Seite befindet sich wie schon in der ersten Ausführung ein Ballon 6. Der Ballon wird durch einen in der Wand des flexiblen Schlauches 1 befindlichen Hohlkanal 17, der an das proximale Ende des Katheters geführt wird, belüftet. Alternativ zu einem einzigen Ballon sind auch unter jeden der Ringe 12 und 13 je ein Ballon zu plazieren.

Am distalen Ring 13 ist distalseitig eine flexible Hohlspitze 13a angebracht, die zur Aufnahme von abgetrennten Material dient.

Innerhalb der Ringe (12,13), die zugleich den Hüllkatheter darstellen, befindet sich der Ringkatheter mit ringförmig angeordneten Lichtleitfasern 8, die einen inneren Hohlkanal 10 umschließen, über dessen distales Ende die Lichtleitfasern 8 leicht hinausstehen. Der Außendurchmesser des Ringkatheters sollte annähernd so groß sein wie der Innendurchmesser der Ringe 12, 13. Der Ringkatheter ist wie auch in der vorbeschriebenen Ausführungsform innerhalb des Hüllkatheters in axialer Richtung beweglich.

Auf der dem Ringkatheter zugewandten Seite des distalen Ringes 13 ist eine Aussparung 18 zur sicheren Aufnahme und Fixierung des vorgeschobenen Ringkatheters vorgesehen.

Die Flexibilität des Gesamtsystems ist variabel und wird dadurch bestimmt wie weit der Ringkatheter im Hüllkatheter vorgeschoben ist. Bei vollkommen vorgeschobenem Ringkatheter ist das System an steifsten.

Mit der beschriebenen flexibleren Ausführungsform zeigen sich in der Praxis weitere Vorteile:
Der Katheter wird mit vorgeschobenem Ringkatheter unter Röntgenkontrolle an der Stelle der Stenose plaziert. An Stellen stärkerer Gefäßwindungen ist der Ringkatheter im Hüllkatheter soweit zurückzuziehen, daß durch die erhöhte Flexibilität des Systems die Passage durch die Gefäßwindung erleichtert oder überhaupt erst ermöglicht wird. Befindet sich der Katheter an der Stelle der Stenose, so wird der Ringkatheter zurückgezogen. Die exakte Plazierung erfolgt unter Sichtkontrolle mit Hilfe des durch den inneren Hohlkanal des Ringkatheters eingeführten Angioskops, bzw. entsprechend unter Ultraschallkontrolle.

Durch Belüftung des Ballons wird der Katheter gegen die Stenose gedrückt. Das Ausmaß des in den Zwischenraum hineinragenden Materials wird unter Sichtkontrolle bzw. Ultraschallkontrolle durch den Inflationsdruck im Ballon gesteuert. Das Stenosematerial wird bei Vorschieben des Ringkatheters mit aktiviertem Laser abgetrennt und verbleibt zunächst im inneren Hohlkanal des Ringkatheters. Anschließend wird das abgetrennte Stenosematerial abgesaugt oder mit einem mechanischen Instrument entfernt, oder in die Hohlspitze geschoben und dort sicher gelagert.

Diese offene Bauweise der Katheteranordnung ermöglicht unter Ultraschalldiagnostik ein wesentlich weiteres Blickfeld als mit vorher beschriebenen Systemen.

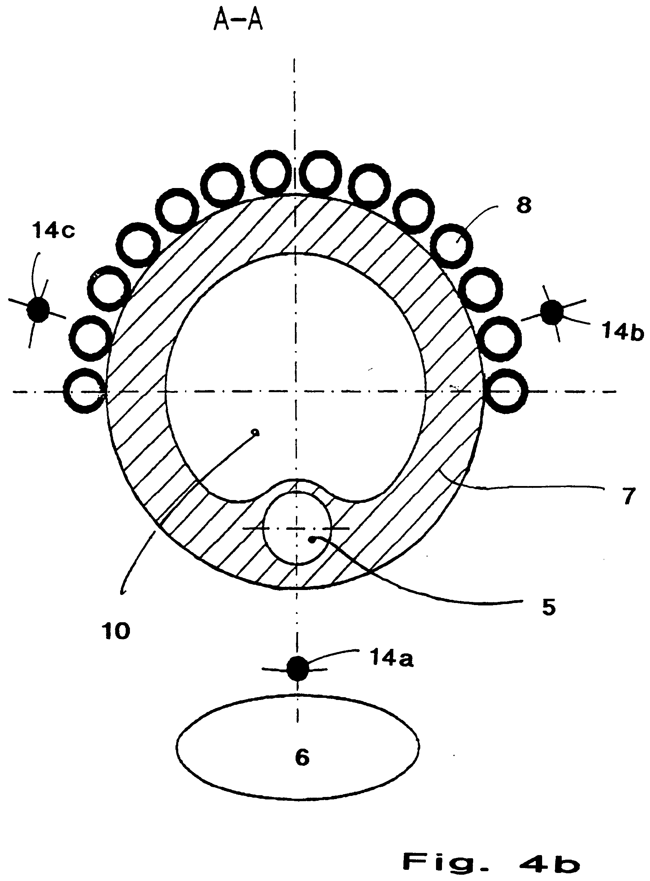

Aus den Figuren 4b bis 4d gehen Schnittzeichnungen hervor, die Schnitte an den, in der Figur 4a angedeuteten Schnittmarkierungen darstellen. Die in den Figuren 4a bis 4f verwendeten Bezugsziffern entsprechen stets den gleichen Bauteilen, so daß diese Figuren in der Zusammenschau zu betrachten sind.

Der Ringkatheter gemäß Figur 4b, besteht, wie bereits beschrieben, aus einem Schlauch 7 mit innerem Hohlkanal 10 zur Aufnahme der Diagnoseeinheit (nicht dargestellt) auf den die Lichtleitfasern 8, die einen Durchmesser von 20 -300 µm haben, aufgebracht sind. Zur Aufnahme des Führungsdrahtes 15 ist ein weiterer innerer Hohlkanal 5 vorgesehen, der im Mantel des Schlauches 7 eingearbeitet ist. Die Verbindungsdrähte 14 a,b,c sind derart frei um den Ringkatheter angeordnet, so daß von oben kommendes Gewebematerial in den Zwischenraum zwischen die Ringanordnung gelangen kann. Mit dem Bezugszeichen 6 ist der aufblasbare Ballon gekennzeichnet.

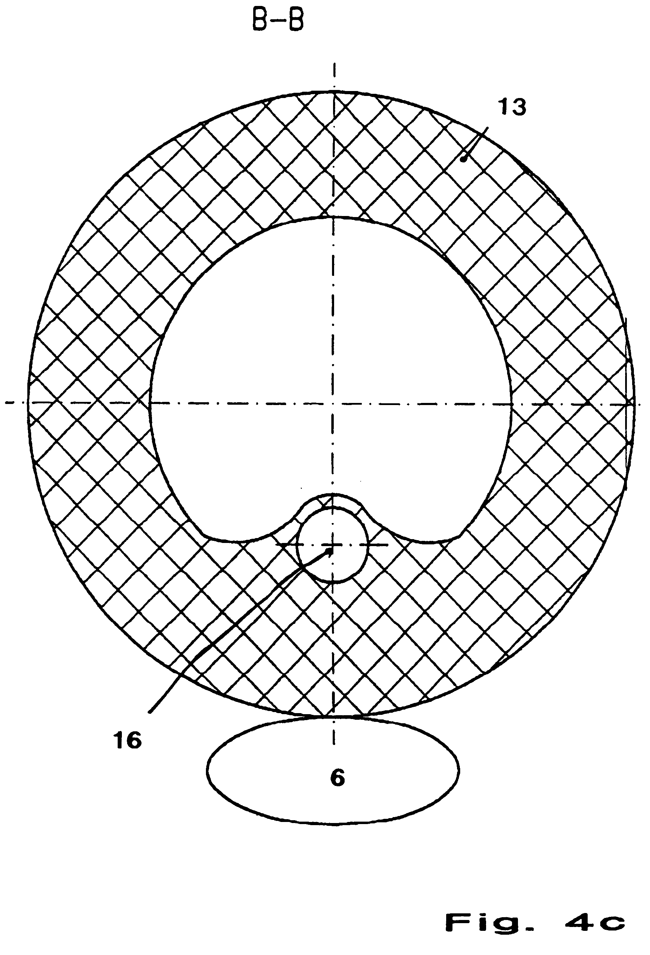

Der distale Ring 13, (Fig.4c, Schnittdarstellung B-B), weist in seiner Wand ebenfalls einen Hohlkanal 5 zur Aufnahme des Führungsdrahtes auf. Deutlich ist aus der Figur 4c das Lumen in der Katheterspitze zu sehen, in das abgetrennte Gewebematerial verbracht werden kann.

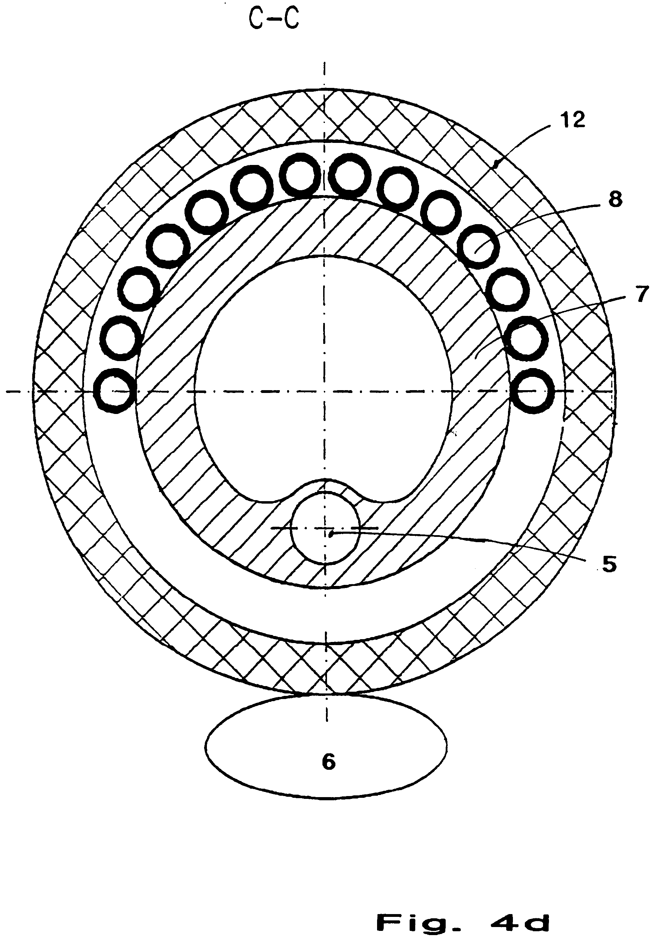

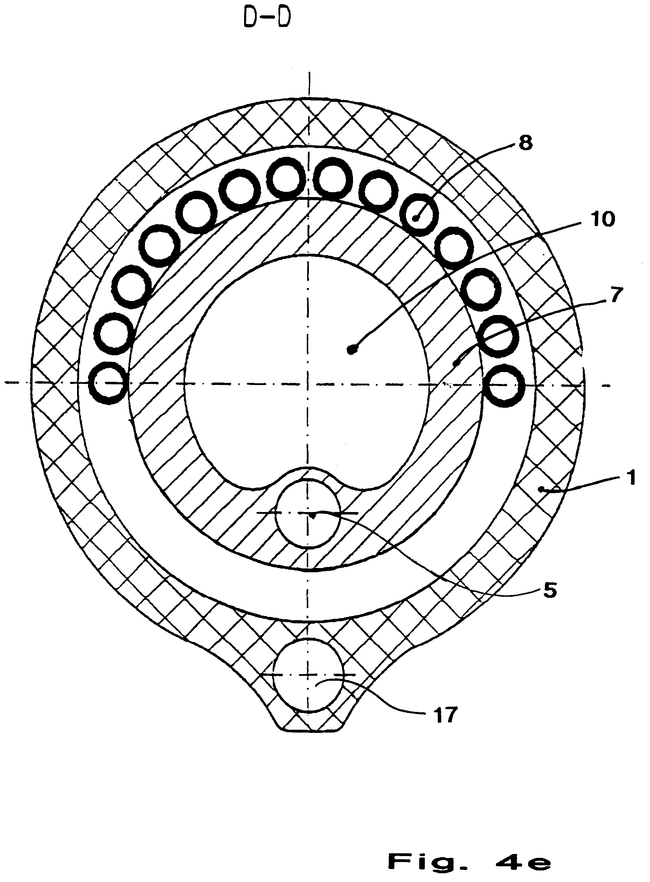

Der innere Durchmesser des proximalen Rings 12 (Fig. 4d, Schnittdarstellung C-C) ist zwecks guter Führung und optimaler Schnittcharakteristik nur wenig größer als der äußere Durchmesser des Ringkatheters, an dessen Aussenumfang die Lichtleitfasern 8 angebracht sind. Vorzugsweise beträgt die Größe des Zwischenraumes zwischen dem Ringkatheter und dem proximalen Ring 12 etwa dem gegenseitigen Abstand zweier Lichtleitfasern. Im unteren Mantelbereich des flexiblen Schlauches 1, (Fig. 4e, Schnittdarstellung D-D) der den proximalen Ring 12 mit den proximalen Enden der Vorrichtung, welche ein Lasergerät und eine diagnostische Auswerteeinheit aufweist, verbindet, befindet sich ein weiterer Hohlkanal 17, durch den der Ballon 6 belüftet werden kann.

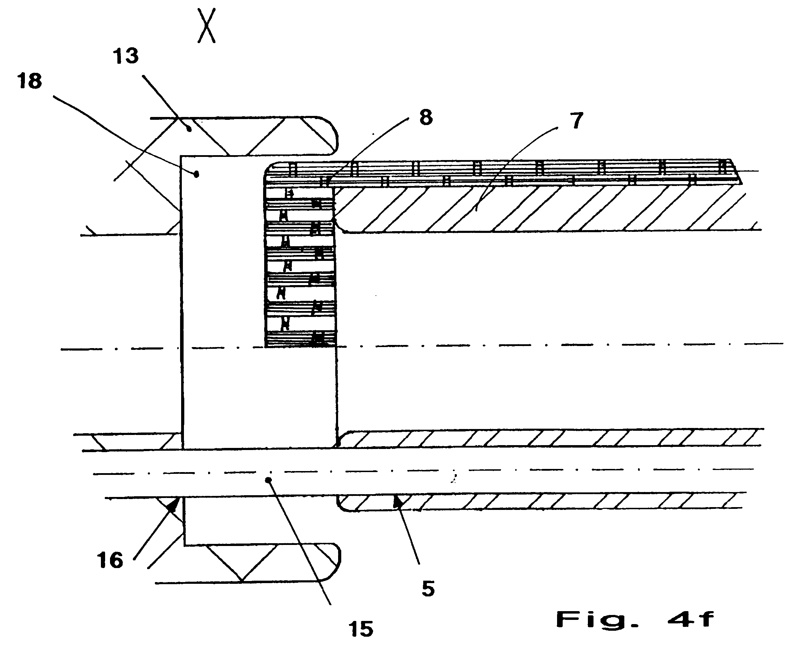

Gemäß Figur 4f (Querschnittsvergrößerung im Bereich X) ist in der dem Ringkatheter zugewandten Seite des distalen Ringes 13 eine Aussparung 18 zur sicheren Aufnahme des vorgeschobenen Faserkatheters vorgesehen. Anhand dieser Querschnittsdarstellung ist zudem der leichte Überstand der Lichtleitfasern 8 gegenüber dem Schlauch 7 zu entnehmen. Durch die gegenseitige, fluchtende Ausrichtung der Hohlkanäle 5 und 16, durch die der Führungsdraht 15 geführt ist, stellt eine exakte Verschiebung des Ringkatheters aus dem Hüllkatheter in den distalen Ring 13 sicher. Abgetrenntes Stenosenmaterial befindet sich sodann innerhalb des Ringkatheters.

### Bezugszeichenliste

- 1: flexibler Schlauch
- 2: Katheterkammer
- 3: Hülse
- 4: Hülsenspitze
- 5: Hohlkanal für Führungsdraht
- 6: Ballon
- 7: flexibler Schlauch
- 8: Lichtleitfasern
- 9: Versteifungsstück
- 10: Innenlumen des Ringkatheters
- 11: Hohlkanal zur Belüftung des Ballons
- 12: proximaler Ring
- 13: distaler Ring
- 14a,b,c: Verbindungsdrähte
- 15: Führungsdraht
- 16: Hohlkanal für Führungsdraht
- 17: Hohlkanal zur Belüftung des Ballons
- 18: Aussparung
- 19: Führungsdraht

## Patentansprüche

1. Vorrichtung zum Abtragen von Gewebematerial in intrakorporalen Hohlkanälen mit einem Laserkatheter, der distalseitig einen Raumbereich vorsieht, in den das abzutragende Gewebematerial einbringbar ist, das mit Hilfe von längs zur Katheterachse beweglichen Lichtleitfasern mittels Lichtapplikation vom übrigen Gewebe abtrennbar ist, die nach dem Abtrennvorgang den Raumbereich abschließen,
dadurch **gekennzeichnet**, daß der Laserkatheter ein Hohlkatheter ist, durch dessen inneren Hohlkanal (10) eine Diagnoseeinheit durchführbar ist.

2. Vorrichtung nach Anspruch 1,
dadurch **gekennzeichnet**, daß die Diagnoseeinheit ein Angioskop ist.

3. Vorrichtung Anspruch 1,
dadurch **gekennzeichnet**, daß die Diagnoseeinheit ein Ultraschallkatheter ist.

4. Vorrichtung nach Anspruch 1,
dadurch **gekennzeichnet**, daß die Diagnoseeinheit ein Laserspektroskopieeinheit ist, bestehend aus zwei Faserbündeln, von denen das eine mit einer Laserquelle verbunden ist, das andere mit einer spektroskopischen Auswerteeinheit.

5. Vorrichtung nach einem der Ansprüche 1 bis 4,
dadurch **gekennzeichnet**, daß durch den inneren Hohlkanal des Hohlkatheters eine Therapieeinheit zur Bearbeitung und Handhabung von Gewebematerial durchführbar ist.

6. Vorrichtung nach einem der Ansprüche 1 bis 5,
dadurch **gekennzeichnet**, daß der Laserkatheter distalseitig wenigstens einen Ballon (6) vorsieht, der über einen Belüftungskanal (11,17) inflatierbar ist.

7. Vorrichtung nach einem der Ansprüche 1 bis 6,
dadurch **gekennzeichnet**, daß der Laserkatheter einen flexiben Schlauch (1) aufweist, in dessen Inneren ein Ringkatheter geführt ist, der auf einem weiteren, flexiblen Schlauch (7) angulär angeordnete Lichtleitfasern (8) vorsieht.

8. Vorrichtung nach einem der Ansprüche 1 bis 7,
dadurch **gekennzeichnet**, daß das distale Ende des Laserkatheters eine Katheterkammer (2) aufweist, die im wesentlichen die Form eines Hohlzylinders hat und etwa bis zur Hälfte ihres Umfanges in axialer Erstreckung eine Ausnehmung vorsieht.

9. Vorrichtung nach Anspruch 8,
dadurch **gekennzeichnet**, daß die Katheterkammer am distalen Ende eine konisch zulaufende Hülsenspitze (14) aufweist.

10. Vorrichtung nach Anspruch 8 oder 9,
dadurch **gekennzeichnet**, daß der Innendurchmesser der Katheterkammer (2) in etwa dem Außendurchmesser des Ringkatheters entspricht, so daß eine Relativbewegung möglich ist.

11. Vorrichtung nach einem der Ansprüche 8 bis 10,
dadurch **gekennzeichnet**, daß der flexible Schlauch (1) und die Katheterkammer (2) einen ineinanderfluchtenden Kanal (5) aufweisen, durch den ein Führungsdraht (15) einführbar ist.

12. Vorrichtung nach einem der Ansprüche 8 bis 11,
dadurch **gekennzeichnet**, daß der flexible Schlauch (1) und die Katheterkammer (2) einen ineinanderfluchtenden Kanal (11) aufweisen, durch den der Ballon inflatierbar ist.

13. Vorrichtung nach einem der Ansprüche 8 bis 12,
dadurch **gekennzeichnet**, daß ein Ballon (6) gegenüber der trapezförmigen Ausnehmnung an der Katheterkammer (2) befestigt ist.

14. Vorrichtung nach einem der Ansprüche 1 bis 7,
dadurch **gekennzeichnet**, daß das distale Ende des Laserkatheters eine Ringanordnung aufweist, die zwei, axial zur Katheterachse beabstandete Ringe (12,13) vorsieht, die über wenigstens zwei, vorzugsweise drei Abstandshalter (14,a,b,c) gegeneinander fixiert sind.

15. Vorrichtung nach Anspruch 14,
dadurch **gekennzeichnet**, daß der proximalseitig angeordnete Ring (12) im wesentlichen den gleichen Querschnitt wie der flexible Schlauch (1) aufweist und mit diesem axial fluchtend fest verbunden ist.

16. Vorrichtung nach Anspruch 14 oder 15,
dadurch **gekennzeichnet**, daß der Ringkatheter und der distalseitig angeordnete Ring (13) einen miteinander fluchtenden, gegenüberliegenden Hohkanal (5,16) aufweisen, durch den ein Führungsdraht (15) einführbar ist.

17. Vorrichtung nach einem der Ansprüche 14 bis 16,
dadurch **gekennzeichner**, daß axial zwischen der Ringanordnung (12,13) der Ringkatheter entlang des Führungsdrahtes (15) bewegbar ist, so daß dieser im ausgefahrenen Zustand eine geschlossene, durchgängige Verbindung zwischen den beiden Ringen (12,13) bildet.

18. Vorrichtung nach einem der Ansprüche 14 bis 17,
dadurch **gekennzeichnet**, daß die Abstandhalter (14a,b,c) stab- oder bandförmig sind.

19. Vorrichtung nach einem der Ansprüche 14 bis 18,
dadurch **gekennzeichnet**, daß der Außendurchmesser des Ringkatheters in etwas dem Innendurchmesser der Ringe (12,13) entspricht.

20. Vorrichtung nach einem der Ansprüche 14 bis 19,
dadurch **gekennzeichnet**, daß der distalseitige Ring (13) an seiner Distalseite eine konisch zulaufende Hülse (13a) vorsieht.

21. Vorrichtung nach einem der Ansprüche 14 bis 20,
dadurch **gekennzeichnet**, daß die Beabstandung der zwei Ringe durch zwei Drähte und einem Band erfolgt.

22. Vorrichtung nach einem der Ansprüche 14 bis 21,
dadurch **gekennzeichner**, daß sich auf der Außenseite der Ringe an der Stelle an der diese mit dem Band verbunden sind ein Ballon (6) befindet, dessen Länge etwa dem Abstand der Ringe (12,13) entspricht.

23. Vorrichtung nach einem der Ansprüche 14 bis 22,
dadurch **gekennzeichnet**, daß sich auf der Außenseite der Ringe an den Stellen an denen diese mit dem Band verbunden sind sich je ein Ballon befindet, dessen Länge etwa der Länge des jeweiligen Ringes entspricht.

24. Vorrichtung nach einem der Ansprüche 14 bis 23,
dadurch **gekennzeichnet**, daß sich ein Hohlkanal zur Belüftung des Ballons (17) innerhalb des flexiblen Schlauchs (1) befindet.

25. Vorrichtung nach einem der Ansprüche 1 bis 24,
dadurch **gekennzeichnet**, daß die Lichtleitfasern (8) des Ringkatheters über den inneren flexiblen Schlauch (7) hinausstehen.

26. Vorrichtung nach einem der Ansprüche 1 bis 25,
dadurch **gekennzeichnet**, daß die Lichtleitfasern (8) des Faserkatheters einen Kerndurchmesser von 20 -300 µm aufweisen.
